(19)

Europäisches
Patentamt
European
Patent Office
Office européen
des brevets

(11) **EP 2 433 262 B1**

(12) **EUROPEAN PATENT SPECIFICATION**

(45) Date of publication and mention
of the grant of the patent:
**27.07.2016 Bulletin 2016/30**

(51) Int Cl.:
***A61B 6/00*** *(2006.01)* ***G06T 7/00*** *(2006.01)*

(21) Application number: **10717241.3**

(86) International application number:
**PCT/IB2010/051454**

(22) Date of filing: **02.04.2010**

(87) International publication number:
**WO 2010/133982 (25.11.2010 Gazette 2010/47)**

(54) **MARKER-FREE TRACKING REGISTRATION AND CALIBRATION FOR EM-TRACKED ENDOSCOPIC SYSTEM**

MARKIERUNGSFREIE VERFOLGUNGSREGISTRATION UND KALIBRATION FÜR EM-VERFOLGTE ENDOSKOPISCHE SYSTEME

ALIGNEMENT DE POURSUITE SANS MARQUEUR ET ÉTALONNAGE POUR SYSTÈME ENDOSCOPIQUE REPÉRÉ ÉLECTROMAGNÉTIQUE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **18.05.2009 US 179031 P**

(43) Date of publication of application:
**28.03.2012 Bulletin 2012/13**

(73) Proprietor: **Koninklijke Philips N.V.**
**5656 AE Eindhoven (NL)**

(72) Inventors:
• **LIU, Xin**
**Briarcliff Manor**
**New York 10510-8001 (US)**
• **GUTIERREZ, Luis Felipe**
**Briarcliff Manor**
**New York 10510-8001 (US)**

(74) Representative: **van Velzen, Maaike Mathilde**
**Philips Intellectual Property & Standards**
**High Tech Campus 5**
**5656 AE Eindhoven (NL)**

(56) References cited:
**WO-A2-2008/065600 JP-A- 2003 265 408**
**JP-K1- 2003 265 408 US-A1- 2005 182 295**
**US-A1- 2007 167 714**

• **GOTTESFELD BROWN L: "A SURVEY OF IMAGE REGISTRATION TECHNIQUES" ACM COMPUTING SURVEYS, ACM, NEW YORK, NY, US, US LNKD- DOI:10.1145/146370.146374, vol. 24, no. 4, 1 December 1992 (1992-12-01), pages 325-376, XP002942558 ISSN: 0360-0300**
• **KENSAKU MORI ET AL: "Hybrid Bronchoscope Tracking Using a Magnetic Tracking Sensor and Image Registration", 1 January 2005 (2005-01-01), MEDICAL IMAGE COMPUTING AND COMPUTER-ASSISTED INTERVENTION - MICCAI 2005 LECTURE NOTES IN COMPUTER SCIENCE;;LNCS, SPRINGER, BERLIN, DE, PAGE(S) 543 - 550, XP019021801, ISBN: 978-3-540-29326-2 * the whole document ***
• **DAISUKE DEGUCHI ET AL: 'A METHOD FOR BRONCHOSCOPE TRACKING USING POSITION SENSOR WITHOUT FIDUCIAL MARKERS' PROCEEDINGS OF SPIE vol. 6511, 08 March 2007, pages 65110N-1 - 65110N-12, XP055061396 DOI: 10.1117/12.709280 ISSN: 0277-786X**
• **Daisuke Deguchi ET AL: "New Image Similarity Measure for Bronchoscope Tracking Based on Image Registration" In: "Field Programmable Logic and Application", 1 January 2003 (2003-01-01), Springer Berlin Heidelberg, Berlin, Heidelberg, XP055061369, ISSN: 0302-9743 ISBN: 978-3-54-045234-8 vol. 2878, pages 399-406, DOI: 10.1007/978-3-540-39899-8_50, * abstract * * section: Feature Value Computation. ***

**(Cont. next page)**

- **KAZUYOSHI ISHITANI ET AL: 'EASY AND STABLE BRONCHOSCOPE CAMERA CALIBRATION TECHNIQUE FOR BRONCHOSCOPE NAVIGATION SYSTEM' PROCEEDINGS OF SPIE vol. 6509, 06 March 2007, pages 65091I-1 - 65091I-12, XP055113405 DOI: 10.1117/12.710398 ISSN: 0277-786X**

**Description**

This disclosure relates to imaging tools, and more particularly to systems and methods for registering and calibrating an endoscope during endoscopic procedures.

Endoscopy is a minimally invasive real-time imaging modality in which a camera is inserted into the body for visual inspection of internal structures such as the lung airways or the gastrointestinal system. Typically, the endoscope is a long flexible fiber-optic system connected to a light source at a proximal end outside of a patient's body and a lens at a distal end inside the patient's body. In addition, some endoscopes include a working channel through which the operator can perform suction or pass instruments such as brushes, biopsy needles or forceps. Video feedback gives a physician or technician cues to maneuver the scope to a targeted region.

Image guided endoscopy, as compared to traditional endoscopy, enjoys an advantage of its real-time connection to a three dimensional (3D) roadmap of a lung while the interventional procedure is performed. It thus has been recognized as a valuable tool for many lung applications. This form of endoscopy requires tracking of the tip of the endoscope in a global coordinate system, in order to associate the location of the endoscope with pre-operative computer tomography (CT) images and display fused images.

In the research of bronchoscope localization, there are three ways to track the tip of the endoscope. Type (a) tracks based on a position sensor mounted to the tip of the endoscope; Type (b) tracks based on live image registration, and Type (c) is a combination of types (a) and (b). Electro-magnetic (EM) guided endoscopy (Type (a) system) has been recognized as a valuable tool for many lung applications, but it requires employing a supplemental guidance device. Although Type (b) is more desirable than Type (a), since it does not employ a supplemental guidance device, constant frame-by-frame registration can be time consuming, and prone to errors, e.g., when fluids inside the airway obscure the video images.

The introduction of an electromagnetic (EM) position sensor to the endoscope (e.g., in Type (a) systems) may overcome this obstacle. In order to provide accurate data fusion between optical images (captured by an endoscope camera) and CT images for an endoscopic procedure, the endoscopic system needs to be calibrated and registered. Calibration refers to the process for determining coordinate offsets between a camera coordinate system and an EM tracker that is attached to the tip of the scope (given the camera intrinsic parameters have already been obtained). Registration refers to determining a coordinate transformation matrix between the EM tracker and the CT image space.

Calibration: In order to integrate data between EM space and camera space, calibration is employed to determine the position and orientation of an EM tracker mounted to the endoscope with respect to the camera coordinates (where the optical axis and center of projection are located). The results of this calibration take the form of six offset constants: three for rotation, three for translation. The goal of calibration in an interventional endoscopic procedure lies in that one can dynamically determine the camera pose based on the EM readings of the attached endoscope tracker.

Generally speaking, calibration is an offline procedure: the calibration parameters can be obtained by imaging an EM-tracked phantom (with a calibration pattern such as a checkerboard) that has known geometric properties, using an EM-tracked endoscope. This involves a cumbersome engineering procedure. Although the desired transformation in this context is between camera coordinates and the endoscope tracker, an array of calibration procedures is needed in each unit of the calibration phantom. For example, a calibration of a pointer tracker, a calibration between a test grid and reference tracker on the grid, a calibration between a camera coordinate and test grid (camera calibration) are all needed to arrive at the destination calibration between the camera coordinate and EM tracker coordinate.

Registration: Another procedure for EM guided endoscopy intervention is to align EM space with pre-operative CT space. Historically, three types of registration methods may be implemented: (1) external fiducial based, (2) internal fiducial based and (3) fiducial-free methods. The advantages and disadvantages of existing registration methods can be found in the following table (Table 1).

**Table 1.** Comparison between different registration approaches.

| Registration Methods | External fiducials | Internal fiducials | Fiducial-free |
|---|---|---|---|
| EM space | Metallic skin markers are placed on the patient's chest before CT scan; These markers remain until after bronchoscopy. | The scope sensor is brought to touch anatomic points such as carina and other branching location | The scope is progressed along medial lines of the air ways. Its position trajectory is continuously recorded. |
| CT space | These markers are identified in CT scans | The corresponding anatomical points in CT were indicated | The midline of the airway is automatically extracted in CT images |

(continued)

| Registration Methods | External fiducials | Internal fiducials | Fiducial-free |
|---|---|---|---|
| Pros | Easy to implement | No external markers, relatively | Dynamic update registration results. |
| Cons | Requires taking a different set of CT scans after skin markers are placed | Have to touch a number of landmark points while the scope is in patient, thus extending the total bronchoscopy time | Assume that the scope moves long the medial line. |

In the fiducial-free registration method cited above, a transformation matrix can be found by minimizing the spatial distance between EM readings from the endoscope tracker, and a midline pathway extracted from the CT images. This means the operator, in order to perform the registration task, has to move steadily along a line to make the data usable for registration. Also, it is unavoidable that when the operator tries to twist the scope toward a sub-branch, or turns the camera aside to examine a wall, the trajectory of the endoscope becomes "off-track" (no longer in the medial line). These data are no longer usable for registration, and have to be discarded until the scope goes back on track (i.e., onto the center line). This data constraint (selectiveness of usable frames) makes real-time registration difficult.

JP 2003 265408 A discloses a method for determining the position of a distal end of an endoscope. Virtual endoscope images of a subject to be tested and information on virtual positions and attitudes of a distal end of a virtual endoscope are stored in a database. When an actual endoscope image of the subject to be tested is obtained, the actual endoscope image is compared with the virtual endoscope images in the database to determine the virtual endoscope image having the highest similarity to the actual endoscope image. The virtual position and attitude stored for this determined highest similarity virtual endoscope image is determined as being the current position and attitude of the distal end of the actual endoscope.

The International Patent Application published as WO 2008/065600 A2 describes a system that fuses real-time Ultrasound images with pre-acquired medical images. The system performs a number of transformation steps to have the respective coordinate systems aligned to each other.

The article "Hybrid Bronchoscope Tracking Using a Magnetic Tracking Sensor and Image Registration" by Kensaku Mori et al., Medical Image Computing and Computer-Assisted Intervention - MICCAI 2005, Lecture Notes in Computer Science, volume 3750, pages 543 to 550 (2005) discloses a hybrid bronchoscope tracking system using a magnetic tracking sensor and image registration. In particular, the position and orientation of a magnetic sensor placed in the working channel of a bronchoscope is provided by a magnetic tracking system. This position and this orientation are used as starting point for an intensity-based registration between real bronchoscopic video images and virtual bronchoscopic images generated from a CT image acquired before the bronchoscopic examination. The transformation obtained by the image registration process provides the position and orientation of the bronchoscope in the CT image.

In accordance with the present principles, a simplified calibration method is provided for circumventing the cumbersome off-line calibration by only computing the offset transformation matrix between camera coordinate and endoscope tracker (given the camera intrinsic parameters have already been obtained). In one embodiment, a fly-through endoluminal view of a passageway (e.g., an airway) is rendered from 3D CT images, or virtual images (e.g., virtual bronchoscopic (VB) images). A software program is configured with an optimization scheme that is capable of identifying a most similar real image (e.g., real bronchoscopic (RB) image) from among a series of candidate real poses to a pre-operative image. A position of an EM position sensor (placed on tip of the endoscope) is determined which is associated with the real image. The position is correlated to the pre-operative image to determine a transformation matrix that indicates how to associate real-time images with the virtual or pre-operative image.

A system that can achieve on-line calibration and marker-free registration is presented. Note that the two procedures are performed independently using the same principal: e.g., the two dimensional image captured by virtual camera and the video image captured by the real camera can be employed and registered to obtain the desired transformation matrices. For the on-tine calibration procedure to be successFully conducted, the registration transformation matrix has to be obtained in advance; likewise, for marker-free registration procedure presented in this context, one has to assume that the calibration matrix is already ready for use. The system is designed to achieve the desired transformation matrix between the EM and the scope camera and between the EM space and CT space intraoperatively. This approach streamlines the data integration procedure for EM-tracked endoscope applications.

The present embodiments may employ image based registration between two-dimensional (2D) video images from an endoscope camera and virtual fly-through endoluminal views derived from CT images with a simple on-line calibration method and a marker-free registration method.

A marker-free registration method is provided for aligning EM space and CT space into coincidence without the operator touching any surface fiducial markers or internal anatomic landmarks. The present principles are operator independent, and do not require a scope touching any external markers or anatomic landmarks to perform the registration. In addition, the scope does not need to be progressed along the middle line or track of the airway.

A system and method for utilizing two-dimensional real-to-virtual image alignment to obtain an EM-to-CT registration matrix and a CT-to-Camera calibration matrix are presented. This includes locating a feature in a pre-operative image and comparing real-time images with the pre-operative image taken of the feature to find a real-time image that closely matches the pre-operative image. A closest match real-time image is registered to the pre-operative image to determine a transformation matrix between a virtual camera pose of the pre-operative image and a real camera pose of the real-time image. This transformation matrix becomes the registration matrix between EM space and CT space (where the calibration matrix is known), becomes the calibration matrix (when the registration matrix is known). The presented system permits marker-free registration and on-line calibration and thus streamlines the data integration procedure for image guided endoscopy applications.

A system and method for image-based registration between images includes locating a feature in a pre-operative image and comparing real-time images taken with a scope with the pre-operative image taken of the feature to find a real-time image that closely matches the pre-operative image. A closest match real-time image is registered to the pre-operative image to determine a transformation matrix between a position of the pre-operative image and a position of the real-time image such that the transformation matrix permits tracking real-time image coordinates in pre-operative image space.

These and other objects, features and advantages of the present disclosure will become apparent from the following detailed description of illustrative embodiments thereof, which is to be read in connection with the accompanying drawings.

This disclosure will present in detail the following description of preferred embodiments with reference to the following figures wherein:

FIG. 1 is a flow diagram showing an illustrative method for image registration in accordance with one embodiment;

FIG. 2 is an illustrative example of a pre-operative virtual image inside a lung airway in accordance with one embodiment;

FIG. 3 is an illustrative diagram depicting an endoscope taking an image at a particular pose associated with the virtual image of FIG. 2;

FIG. 4 is an illustrative diagram showing coordinate systems for a camera, a tracker and a virtual image space in accordance with the present principles;

FIG. 5 is an illustrative diagram showing matching between a pre-operative image and a video real-time image in accordance with the present principles;

FIG. 6 is a flow diagram showing a method for image-based registration between video and pre-operative images in accordance with one embodiment;

FIG. 7 is a block diagram showing a system for image-based registration between video and pre-operative images in accordance with the present principles;

FIG. 8 is an illustrative diagram showing a system for an on-line calibration with fiducial-based registration using a phantom reference in accordance with the present principles; and

FIG. 9 is a flow diagram showing a method for on-line calibration for guided endoscopy in accordance with another embodiment.

The present disclosure describes systems and methods for scope calibration and registration. A simple method for calibrating an electro-magnetic (EM) guided endoscopy system computes a transformation matrix for an offset between a camera coordinate and an endoscope tracker. The offset distance between a camera frame and an endoscope tracker frame is reflected in a disparity in 2D projection images between a real video image and a virtual fly-through image. Human eyes or a computer are used to differentiate this spatial difference and rebuild the spatial correspondence. The spatial offset becomes the calibration result.

An endoscopy system and method use marker-free, image-based registration, matching a single 2D video image from a camera on the endoscope with a CT image or other virtual image, to find a transformation matrix between CT space and EM (electromagnetic tracking) space. The present embodiments (in the form of a bronchoscope, for example) may include: (1) an EM position sensor placed on a tip of the bronchoscope, (2) reconstructed virtual bronchoscopic (VB) images from CT scans (or other technology, e.g., MRI, sonogram, etc.) and (3) software with an optimization scheme to identify the most similar-to-VB real bronchoscopic (RB) image among on a series of candidate RB poses. Progression of the bronchoscope only along a middle line of an airway is not required. Markers on or in the patient are not required. The system and method are operator independent, and do not require a scope's touching any external markers or anatomic landmarks, to perform the registration.

In particularly useful embodiments, the scope may include a bronchoscope or any scope for pulmonary, digestive system, or other minimally invasive surgical viewing. In other embodiments, an endoscope or the like is employed for other medical procedures as well. These procedures may include minimally invasive endoscopic pituitary surgery, endoscopic skull base tumor surgery, intraventricular neurosurgery, arthroscopic surgery, laparoscopic surgery, etc. Other scoping applications are also contemplated.

It should be understood that the present invention will be described in terms of a bronchoscope; however, the teachings of the present invention are much broader and are applicable to any optical scope that can be employed in internal viewing of branching, curved, coiled or other shaped systems (e.g., digestive systems, circulatory systems, piping systems, passages, mines, caverns, etc.). Embodiments described herein are preferably displayed for viewing on a display monitor. Such monitors may include any suitable display device including but not limited to handheld displays (e.g., on personal digital assistants, telephone devices, etc.), computer displays, televisions, designated monitors, etc. Depending of the scope, the display may be provided as part of the system or may be a separate unit or device. Further, virtual images may be generated using CT scanning technology although other imaging technology may also be employed such as for example, sonograms, magnetic resonance images, computer generated images, etc.

It should also be understood that the optical scopes may include a plurality of different devices connected to or associated with the scope. Such devices may include a light, a cutting device, a brush, a vacuum line, a camera, etc. These components may be formed integrally with a head on a distal end portion of the scope. The optical scopes may include a camera disposed at a tip of the scope or a camera may be disposed at the end of an optical cable opposite the tip. Embodiments may include hardware elements, software elements or both hardware and software elements. In a preferred embodiment, the present invention is implemented with software, which includes but is not limited to firmware, resident software, microcode, etc.

Furthermore, the present principles can take the form of a computer program product accessible from a computer-usable or computer-readable medium providing program code for use by or in connection with a computer or any instruction execution system. A computer-usable or computer readable medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device). Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W) and DVD.

A data processing system suitable for storing and/or executing program code may include at least one processor coupled directly or indirectly to memory elements through a system bus. The processor or processing system may be provided with the scope system or provided independently of the scope system. The memory elements can include local memory employed during actual execution of the program code, bulk storage, and cache memories which provide temporary storage of at least some program code to reduce the number of times code is retrieved from bulk storage during execution. Input/output or I/O devices (including but not limited to keyboards, displays, pointing devices, etc.) may be coupled to the system either directly or through intervening I/O controllers.

Network adapters may also be coupled to the system to enable the data processing system to become coupled to other data processing systems or remote printers or storage devices through intervening private or public networks. Modems, cable modem and Ethernet cards are just a few of the currently available types of network adapters.

In accordance with the present principles, three local coordinate systems need to be inter-connected to permit a mapping of events therebetween. These include a camera coordinate system (where the center of projection and optical axis are located), EM sensor coordinate system, and CT coordinate system.

$$p_{CT} = \mathrm{T}^{CT}_{Cam} p_{Cam} = \mathrm{T}^{CT}_{EM} \mathrm{T}^{EM}_{Cam} p_{Cam} \quad (1)$$

where $p_{ct}$ is a position (pose) in CT space, and $p_{cam}$ is a position (pose) in camera space. Ultimately, one needs to identify the relationship $\mathrm{T}^{CT}_{Cam}$ (transformation between CT space and camera space) to use a pre-operative CT roadmap to guide an endoscopic procedure. Matrix $\mathrm{T}^{EM}_{Cam}$ is the calibration matrix between the EM sensor on the tip of the endoscope and the camera coordinate system, matrix $\mathrm{T}^{CT}_{EM}$ is the registration matrix between EM and CT spaces.

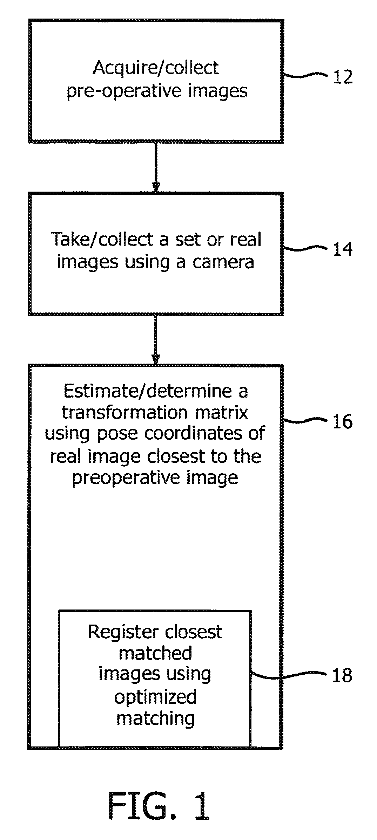

Referring now to the drawings in which like numerals represent the same or similar elements and initially to FIG. 1, a method is shown to seek out the transformation $\mathrm{T}^{CT}_{Cam}$. This is performed by acquiring one pre-operative image (e.g., a CT image) in block 12. The pose of the pre-operative position will be recorded as $P_v$. A set of real images are taken using the camera on an endoscope in block 14. The real images are close to some landmark position, such as, e.g., a first branching position (e.g., the carina in the lungs). The operator will move the endoscope close enough to match the pre-operative image. When satisfied with the pose of the scope, the operator can start to acquire a series of images from pose $P_{i-N}$ to $P_{i+N}$ (for initial pose position $P_i$).

A transformation matrix is estimated in block 16 by seeking out the pose of a camera which renders a real image most similar to the pre-operative image. In block 18, a mutual-information based registration method can be employed to find the most similar image whose pose is denoted as $P_R$. The transformation matrix between $P_V$ and $P_R$ becomes the desired registration result and can be used to track real image space to pre-operative image space.

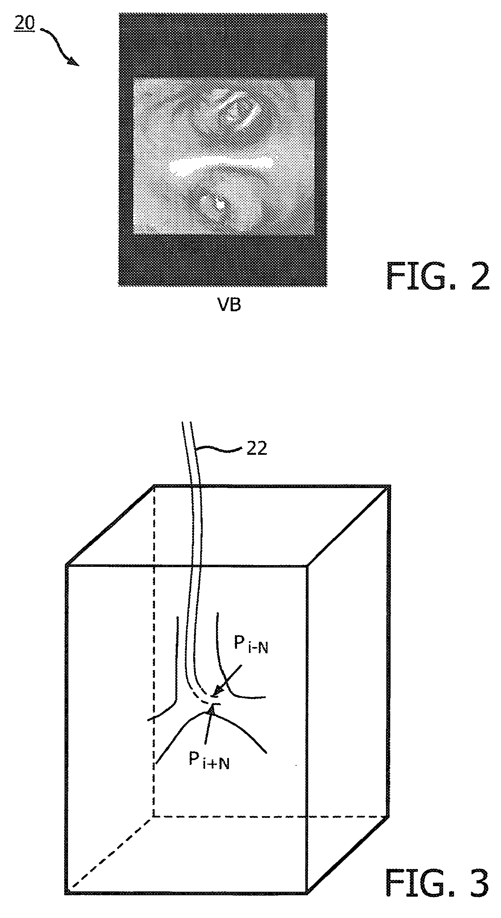

Referring to FIGS. 2 and 3, a virtual image 20 is shown at a carina position of a lung. A camera pose at the virtual position (VB) is recorded as $P_V$. The operator moves an endoscope 22 with a camera for collecting images close enough to match the image VB. The VB camera pose is known and stored in memory. When the operator is satisfied with the pose of the scope, the operator can start to acquire a series of images from pose $P_i$ to $P_{i+N}$ (or from $P_{i-N}$). A mutual-information based registration method will be employed to find the most similar image whose pose is denoted as $P_R$. The camera pose $P_R$ corresponds to the best match between VB and the selected RB. The transformation matrix between $P_V$ and $P_R$ is constructed and becomes the desired registration result. Image similarity may be determined using computer implemented software tools or may be performed by a human operator depending on the circumstances.

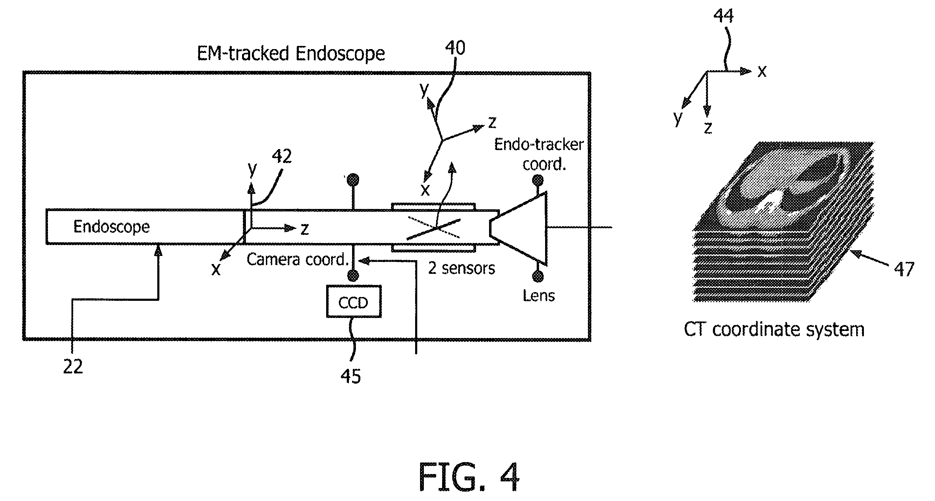

Referring to FIG. 4, a relationship between an EM tracker coordinate system 40, a camera coordinate system 42 and a CT coordinate system 44 is illustratively depicted. The three local coordinate systems 40, 42 and 44 need to be interconnected to permit transformation between the camera coordinate system 42 (where the center of projection and optical axis are located), EM sensor coordinate system 40, and CT coordinate system 44. This can be expressed as set forth in Eq. (1). One needs to identify the relationship $T_{Cam}^{CT}$ (transformation between CT space and camera space) to use a pre-operative CT roadmap to guide an endoscopic procedure. In one embodiment, registration is employed to align EM with CT space to obtain $T_{EM}^{CT}$. $T_{Cam}^{EM}$ is the calibration matrix between the EM sensor on the tip of the endoscope and the camera coordinate system. This can be determined through a calibration procedure. In accordance with one aspect of the present principles, a method is provided to obtain $T_{EM}^{CT}$ Eq. (2)) that otherwise can only be acquired via a fiducial-based method.

$$T_{EM}^{CT} = T_{Cam}^{CT} T_{EM}^{Cam} \qquad (2)$$

Transformation, $T_{Cam}^{CT}$, is estimated by finding the pose of a given captured VB, and seeking out the pose of a camera which renders a real image most similar to the virtual image.

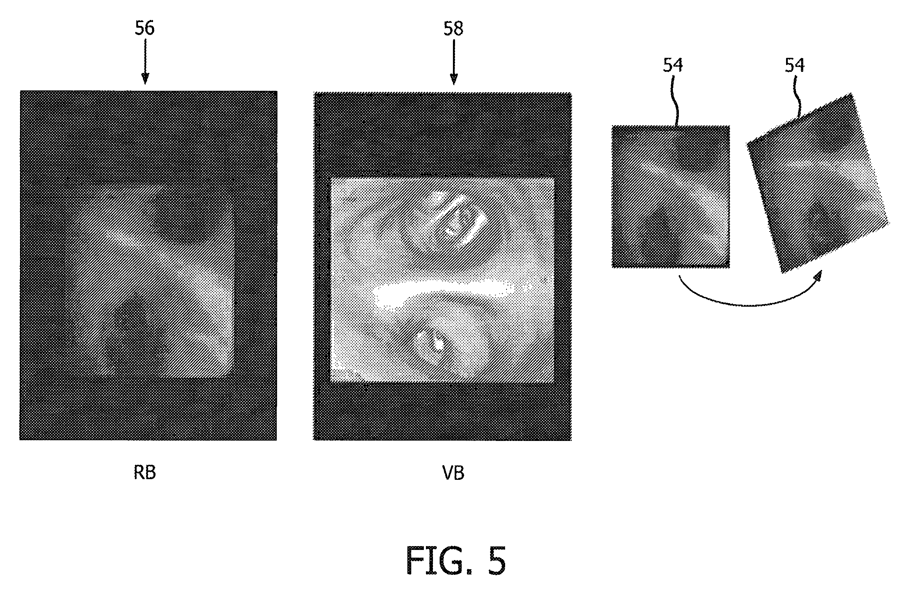

A human operator only needs to bring the scope close enough to the VB pose by examining and comparing the similarities between VB and RB images. Then, a number of RB frames will be collected in a neighborhood centered on an initialization point $P_i$ (e.g., from pose $P_{i-N}$ to $P_{i+N}$ in FIG. 3). The registration between RB and VB is done by maximizing the normalized mutual information (NMI) between the video taken by a CCD camera 45 (RB images) or the like and virtual images (in CT space 47). The use of an iterative optimization technique can be used to identify this local maximum (see FIG.5).

Referring to FIG. 5, a number of real (RB) images 56 are collected, and they are compared to a virtual or pre-collected (VB) image 58 until maximum similarity has been found. Then, the images are registered by moving the images (54) with respect to each other. This movement is stored in a matrix and provides a one-time transformation for relating respective coordinate systems. The present embodiments can be applied to any EM-tracked endoscopic system that uses registration between, e.g., pre-operative CT space with EM tracking space (real video images).

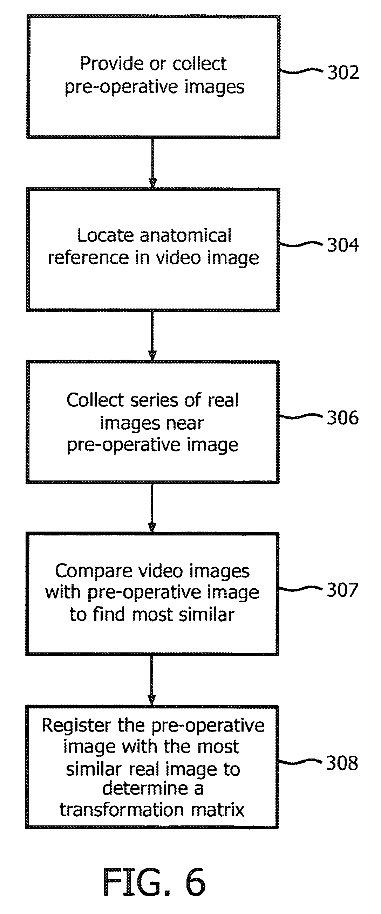

Referring to FIG. 6, a method for image-based registration between images is illustratively shown in accordance with one illustrative embodiment. In block 302, computer tomography (CT) (or other pre-operative) images of a subject are collected or provided. Advantageously, no markers are needed in the CT images. In block 304, an anatomical reference or feature is located in a video image (e.g., a real-time image taken with a camera of an endoscope) which corresponds to a particular pre-operative image. This may include tracking an endoscope with electromagnetic tracking.

In block 306, a series of video images are collected around the feature to attempt to replicate the pose of the virtual or pre-operative image. Then, in block 307, the video images are compared with the CT image to find a closest match between the video image and the CT image. This may include optimizing the matching procedure to find a maximum similarity between images to determine the closest matched real image to the CT image. In block 308, the video image is registered to the CT match image using pose positions associated with the real image matched with the CT image to create a transformation matrix based upon the rotations and translations needed to align the poses of the tracker with the pre-operative image pose. The transformation matrix between the CT space and image tracking space is determined and is based solely on image registration. The method is operator independent and free of any external markers or anatomic landmarks which need to be contacted by a tracker for registration. The transformation matrix is employed to register coordinates of the CT images to electromagnetic tracking coordinates during an endoscopic procedure. The endoscope progression may be other than along a middle line of a passage being observed.

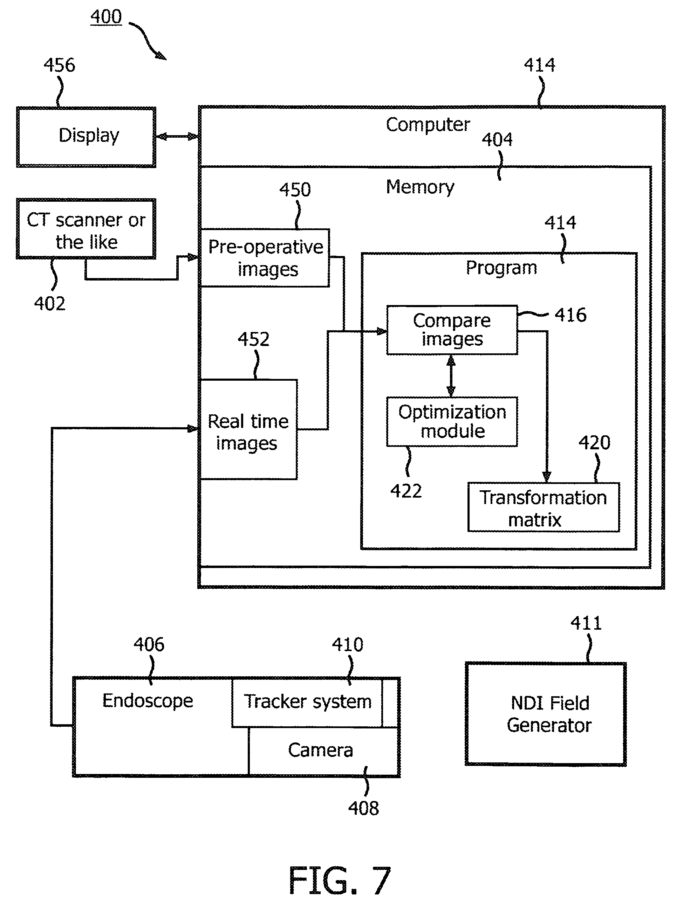

Referring to FIG. 7, a system 400 for image-based registration between images is illustratively shown. The system 400 includes a computer tomography (CT) scanner 402 (or other pre-operative imager or scanner) although the scanner 402 is not needed as the CT images may be stored in memory 404 and transferred to the system 400 using storage media or network connections. The memory 404 and/or scanner are employed to store/collect CT images of a subject, such as a patient for surgery. An endoscope 406 includes a camera 408 for collecting real-time images during a procedure. The endoscope 406 includes a tracker system 410, e.g., an electromagnetic (EM) tracker for locating a tip of the endoscope. The tracker system 410 needs to have its coordinate system mapped or transformed into the CT coordinate system. The tracker system 410 employs an NDI field generator 411 to track the progress of the endoscope 406.

A computer implemented program 412 is stored in memory 404 of a computer device 414. The program 412 includes a module 416 configured to compare a real-time video image 452 taken by the camera 408 with CT images 450 to find a closest match between the real-time images and the CT image. The program 412 includes an optimization module 422 configured to find a maximum similarity to determine the closest match CT image. The program 412 is configured to register a closest matched real-time image to a pre-operative image in CT space to find a transformation matrix 420 between the CT space and image tracking space such that the transformation matrix 420 is based solely on image registration, is operator independent, and free of any external markers or anatomic landmarks to perform the registration. The transformation matrix 420 is employed to register coordinates of the CT images to electromagnetic tracking coordinates during an endoscopic procedure. A display 456 may be employed to view the real-time and/or virtual/pre-operative images during the procedure. The display 456 is configured to show endoscope progression in pre-operative image space. The marker-free registration process assumes a calibration process is employed beforehand to determine the relationship between the camera coordinate system and the tracking (EM) coordinate system.

In accordance with another embodiment, an approach is provided which uses registration to provide a calibration. The registration in this embodiment includes any type of registration including fiducial marker registration. Calibration includes a calibration matrix and may also include camera or other parameter calibrations (e.g., focal length, etc.). An offset distance between a camera frame and an endoscope tracker frame is reflected in the disparity in 2D projection images between a real video image and a virtual fly-through image (from CT scans). Human eyes and computers have the capability to differentiate these spatial differences and rebuild the spatial correspondence.

The present principles include making use of (1) an EM tracking system, (2) a phantom with EM trackable fiducials on a surface, (3) a mechanical arm (optional) that holds and stabilizes an endoscope, (4) a computer with software that collects before and after poses of an endoscope tracker, (5) input from the stereo sense of a human operator to match a real endoscopic (for example, a bronchoscopic) image (RB) with a virtual endoscopic (bronchoscopic) image (VB), and (6) software that runs an optimization scheme to find the maximum similarity between the VB and RB images.

The data integration procedure is streamlined because a same phantom, designed for fiducial-based registration, is used for both calibration and registration tasks. A calibration procedure which is independent of camera calibration (the estimation of internal and external parameters of the camera) is achieved.

Using an image-based method, an on-line calibration method is presented given that an EM-CT registration matrix has already been acquired. In this case, the fiducial-based registration method is first employed to register images between CT space and EM tracked endoscopy. The registration brings the CT coordinates and tracker coordinates into coincidence.

Referring again to FIG. 5, fine adjustment of the EM-tracked endoscope for matching the real bronchoscopic image (RB) 56 with the virtual bronchoscopic image (VB) 58 is conducted. This results in a desired calibration matrix by computing before and after endoscope tracker poses. The spatial offset between them becomes the calibration result in this case (as opposed to the registration result, as described earlier).

In FIG. 5, RB 56 is a real bronchoscopic video image and VB 58 is a virtual bronchoscopic image reconstructed from CT data. Note that RB 56 and VB 58 may have been registered previously via the fiducial based approach (or other method). RB 56 and VB 58 present a small spatial displacement. An operator will adjust (54) the scope until RB 56 matches with the VB 58 more closely. A number of RB frames are compared to VB 58 using an optimization scheme until maximum similarity has been found. This yields a calibrated RB 54. From this example, the endoscope will probably need to rotate anti-clockwise and retreat backward. The tracking data of the endoscope will be recorded before and after the adjustment.

Relationships between an EM sensor coordinate system and a camera coordinate system provide calibration while registration couples a CT coordinate system to the EM sensor coordinate system and the camera coordinate system. The three local coordinate systems use inter-registrations to track positions between them. One needs to identify

the relationship $\mathrm{T}_{Cam}^{CT}$ (Eq. (2)) to associate a pre-operative CT roadmap with intraoperative endoscopic videos. Fiducial based registration is a process that may be employed to align EM space with CT space and arrive at the transformation matrix $\mathrm{T}_{EM}^{CT}$.

Usually after fiducial based registration, CT and EM frames are largely aligned. These frames however may present a small spatial displacement owing to the unknown $\mathrm{T}_{Cam}^{EM}$. (E.g., EM sensor on the tip of the endoscope is uncalibrated with the camera coordinate system).

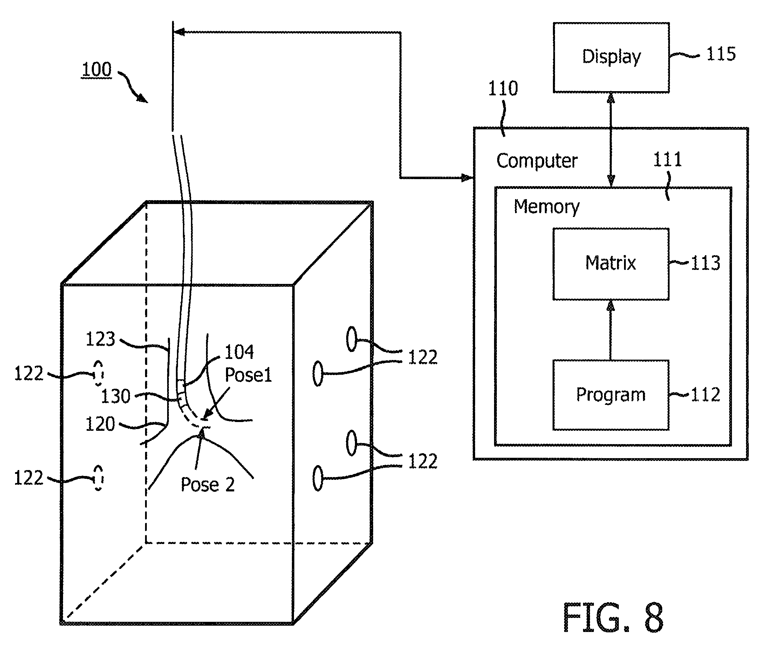

Referring to FIG. 8, in accordance with the present principles, an on-line calibration system 100 includes a computer 110 having software 112 that collects the before and after poses of an endoscope tracker 104. A stereo sense of the human operator or computer program 112 is provided to determine discrepancies between images. The software program 112 runs an optimization scheme to find the maximum similarity between virtual and real images. This may be performed by frame by frame comparisons using known image analysis software.

A computer tomography (CT) scanner (not shown) may be configured to collect pre-operative CT images (or other technology for generating, collecting and storing a virtual map or images) of a subject having fiducial markers 122. The pre-operative images may be stored in memory 111 and transferred to the system 100 using storage media or network connections. The memory 111 and/or scanner are employed to store/collect CT images of a subject, such as a patient for surgery. The endoscope 108 includes a camera 130 for collecting real-time images during a procedure. The endoscope 108 includes an electromagnetic tracker 104 for locating the tip of the endoscope 108.

A phantom reference 120 is employed for assisting in registering pre-operative scan images to EM tracked positions. By touching each of the markers 122 using the tracker device 104, the CT image and is registered to EM tracked positions obtained by the tracker 104. A calibrated pointer-tracker (EM tracker 104) is used to touch each of the surface fiducials 122, so that a point-based registration aligns the CT space with the EM position ( $\mathrm{T}_{EM}^{CT}$ ) such that when the tracker on the endoscope is advanced in the airway, the pre-operative or CT (VB) images will update together with the real (RB) images. The lung phantom 120 is employed to perform dual roles to assist in calibration and registration.

For calibration, the endoscope 108 is inserted into the bronchus 123 and using the lung phantom 120, which has a few surface fiducials 122, a position is determined to perform the calibration. At the position, a real image (RB) and a closest corresponding CT image (VB) are provided (a VB image at pose 1 will be determined or captured). Due to a slight displacement between the VB and the RB images, the operator will adjust the scope 108 until the RB matches with the VB more closely. This yields a calibrated RB (at pose 2). Pose 1 refers to the RB pose after fiducial-based registration and pose 2 refers to the calibrated RB pose with the VB image. The RB video image from pose 2 matches most closely with the VB image. The rotation and translation matrix from pose 2 to pose 1 becomes the targeted calibration result. From the example in FIG. 5, the endoscope 108 may need an anti-clockwise rotation together with a slight backward retraction. The tracking data of the endoscope 108 will be recorded before and after the adjustment.

Computer device 110 and its memory 111 store the rotation and translation information in a matrix 113 for calibrating the tracker 104 to a camera image by adjusting the endoscope 108 until the image obtained by a camera 130 associated with the tracker 104 matches with the registered CT image as described. The rotation and translation matrix 113 is employed to calibrate coordinates of the camera 130 to the tracker 104. A display 115 may be employed to view the real-time and/or virtual images during the procedure.

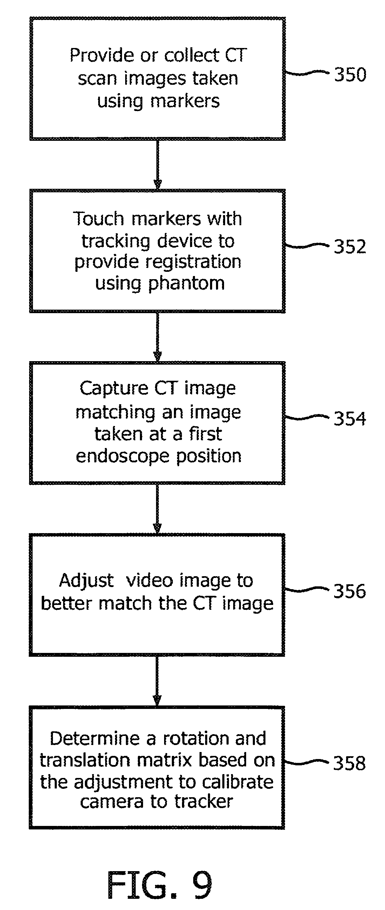

Referring to FIG. 9, a method for on-line calibration for endoscopy is illustratively shown in accordance with one exemplary embodiment. In block 350, computer tomography (CT) images (or virtual images generated from a different technology) of a subject having markers are collected or provided. In block 352, a tracker device is contacted with (e.g., touches) each of the markers to register the CT image and an image obtained by the tracker to obtain, e.g., a fiducial-based registration.

In block 354, a real image is captured with an endoscope at a first position. In block 356, the endoscope is adjusted until the image obtained by a camera matches with a CT image of the same region at a second position. Adjusting the scope may include adjustment by an operator.

In block 358, a rotation and translation matrix is determined to calibrate the tracker based on the motion made during the adjustment stage (block 356). The rotation and translation matrix is employed to calibrate coordinates of a camera to the tracker such that the CT images will update together with the real-time images.

In interpreting the appended claims, it should be understood that:

a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function; and

e) no specific sequence of acts is intended to be required unless specifically indicated.

Having described preferred embodiments for systems and methods (which are intended to be illustrative and not limiting), it is noted that modifications and variations can be made by persons skilled in the art in light of the above teachings. It is therefore to be understood that changes may be made in the particular embodiments of the disclosure disclosed which are within the scope of the embodiments disclosed herein as outlined by the appended claims.

**Claims**

1.  A method for image-based registration between images, comprising:

    comparing real-time images taken with a camera (130) of a tracked endoscope (108) with a pre-operative image for a same subject, which has been registered with a tracker (104) for tracking the endoscope (108), to find a closest match between the real-time images and the pre-operative image, wherein the pre-operative image is a two dimensional virtual camera image;
    registering the closest match real-time image to the pre-operative image to determine a transformation matrix between a position of the pre-operative image and a position of the real-time image;
    determining a transformation between the tracker (104) and a coordinate system of the camera (130) based on the transformation matrix between the position of the pre-operative image and the position of the real-time image and based on an already acquired registration of the pre-operative image and the tracker (104).

2.  The method as recited in claim 1, wherein the real-time images are collected using an endoscope camera (130) with electromagnetic tracking.

3.  The method as recited in claim 2, further comprising progressing the endoscope camera (130) other than along a middle line of a passage being observed.

4.  The method as recited in claim 1, further comprising determining a virtual camera pose for the pre-operative image and correlating the pre-operative pose with a real camera pose of the closest matched real image to determine the transformation matrix.

5.  A system for image-based registration between images, comprising:

    an endoscope (108) including a camera (130) for collecting real-time images during a procedure, the endoscope including a tracker (104) for locating a tip of the endoscope (108); and
    a computer implemented program (112) stored in memory media, configured to:

    compare a set of real-time images taken by the camera (130) with a pre-operative image registered with the tracker (104) for a same subject to find a closest match between the real-time images and the pre-operative image, wherein the pre-operative image is a two dimensional virtual camera image,
    register the closest match real-time image to the pre-operative image to determine a transformation matrix between a position of the pre-operative image and a position of the real-time image,
    determine a transformation between the tracker (104) and a coordinate system of the camera (130) based on the transformation matrix between the position of the pre-operative image and the position of the real-time image and based on an already acquired registration of the pre-operative image and the tracker (104).

6.  The system as recited in claim 5, wherein endoscope progression is tracked other than along a middle line of a passage being observed.

**Patentansprüche**

1.  Verfahren zur bildbasierten Registrierung zwischen Bildern, das Folgendes umfasst:

    Vergleichen von mit einer Kamera (130) aufgenommenen Echtzeitbildern eines verfolgten Endoskops (108) mit einem präoperativen Bild für einen gleichen Patienten, das mit einem Tracker (104) zum Verfolgen des Endoskops (108) registriert wurde, um eine beste Übereinstimmung zwischen den Echtzeitbildern und dem präoperativen Bild zu finden, wobei das präoperative Bild ein zweidimensionales virtuelles Kamerabild ist; Registrieren des am besten übereinstimmenden Echtzeitbilds mit dem präoperativen Bild, um eine Transformationsmatrix zwischen einer Position des präoperativen Bilds und einer Position des Echtzeitbilds zu ermitteln; Ermitteln einer Transformation zwischen dem Tracker (104) und einem Koordinatensystem der Kamera (130) basierend auf der Transformationsmatrix zwischen der Position des präoperativen Bilds und der Position des Echtzeitbilds und basierend auf einer bereits erfassten Registrierung der präoperativen Bilds und des Trackers (104).

2. Verfahren nach Anspruch 1, wobei die Echtzeitbilder unter Verwendung einer Endoskopkamera (130) mit elektromagnetischer Verfolgung aufgenommen werden.

3. Verfahren nach Anspruch 2, weiterhin umfassend das Vorschieben der Endoskopkamera (130) auf andere Weise als entlang einer Mittellinie einer beobachteten Passage.

4. Verfahren nach Anspruch 1, weiterhin umfassend das Ermitteln einer virtuellen Kamerapose für das präoperative Bild und das Korrelieren der präoperativen Pose mit einer echten Kamerapose des am besten übereinstimmenden Bilds, um die Transformationsmatrix zu ermitteln.

5. System zur bildbasierten Registrierung zwischen Bildern, das Folgendes umfasst:

ein Endoskop (108) mit einer Kamera (130) zum Aufnehmen von Echtzeitbildern während einer Prozedur, wobei das Endoskop einen Tracker (104) zum Lokalisieren einer Spitze des Endoskops (108) umfasst; und ein computer-implementiertes Programm (112), das in Speichermedien gespeichert ist und konfiguriert ist zum:

Vergleichen eines durch die Kamera (130) aufgenommenen Satzes von Echtzeitbildern mit einem mit dem Tracker (104) registrierten präoperativen Bild für einen gleichen Patienten, um eine beste Übereinstimmung zwischen den Echtzeitbildern und dem präoperativen Bild zu finden, wobei das präoperative Bild ein zweidimensionales virtuelles Kamerabild ist;

Registrieren des am besten übereinstimmenden Echtzeitbilds mit dem präoperativen Bild, um eine Transformationsmatrix zwischen einer Position des präoperativen Bilds und einer Position des Echtzeitbilds zu ermitteln; Ermitteln einer Transformation zwischen dem Tracker (104) und einem Koordinatensystem der Kamera (130) basierend auf der Transformationsmatrix zwischen der Position des präoperativen Bilds und der Position des Echtzeitbilds und basierend auf einer bereits erfassten Registrierung der präoperativen Bilds und des Trackers (104).

6. System nach Anspruch 5, wobei das Vorschieben des Endoskops auf andere Weise als entlang einer Mittellinie einer beobachteten Passage verfolgt wird.

**Revendications**

1. Procédé pour l'alignement basé sur des images entre des images, comprenant :

la comparaison d'images en temps réel prises avec une caméra (130) d'un endoscope suivi (108) à une image préopératoire pour un même sujet, qui a été alignée avec un suiveur (104) destiné à suivre l'endoscope (108), pour trouver une correspondance très étroite entre les images en temps réel et l'image préopératoire, dans lequel l'image préopératoire est une image de caméra virtuelle bidimensionnelle ; l'alignement de l'image en temps réel à correspondance très étroite sur l'image préopératoire afin de déterminer une matrice de transformation entre une position de l'image préopératoire et une position de l'image en temps réel ; la détermination d'une transformation entre le suiveur (104) et un système de coordonnées de la caméra (130) sur la base de la matrice de transformation entre la position de l'image préopératoire et la position de l'image en temps réel et sur la base d'un alignement déjà acquis de l'image préopératoire et du suiveur (104).

2. Procédé selon la revendication 1, dans lequel les images en temps réel sont collectées en utilisant une caméra d'endoscope (130) avec un suivi électromagnétique.

3. Procédé selon la revendication 2, comprenant en outre la progression de la caméra d'endoscope (130) ailleurs que le long d'une ligne centrale d'un passage observé.

4. Procédé selon la revendication 1, comprenant en outre la détermination d'une pose de caméra virtuelle pour l'image préopératoire et la corrélation de la pose préopératoire avec une pose de caméra réelle de l'image réelle à correspondance très étroite afin de déterminer la matrice de transformation.

5. Système pour l'alignement basé sur des images entre des images, comprenant :

un endoscope (108) incluant une caméra (130) pour collecter des images en temps réel durant une procédure, l'endoscope incluant un suiveur (104) pour localiser une pointe de l'endoscope (108) ; et
un programme exécuté par ordinateur (112) stocké dans des supports de mémoire, configuré pour :

comparer un ensemble d'images en temps réel prises par la caméra (130) à une image préopératoire alignée avec le suiveur (104) pour un même sujet pour trouver une correspondance très étroite entre les images en temps réel et l'image préopératoire, dans lequel l'image préopératoire est une image de caméra virtuelle bidimensionnelle,
aligner l'image en temps réel à correspondance très étroite sur l'image préopératoire pour déterminer une matrice de transformation entre une position de l'image préopératoire et une position de l'image en temps réel,
déterminer une transformation entre le suiveur (104) et un système de coordonnées de la caméra (130) sur la base de la matrice de transformation entre la position de l'image préopératoire et la position de l'image en temps réel et sur la base d'un alignement déjà acquis de l'image préopératoire et du suiveur (104).

6. Système selon la revendication 5, dans lequel la progression d'endoscope est suivie ailleurs que le long d'une ligne centrale d'un passage observé.

Acquire/collect
pre-operative images

— 12

Take/collect a set or real
images using a camera

— 14

Estimate/determine a
transformation matrix
using pose coordinates of
real image closest to the
preoperative image

— 16

Register closest
matched
images using
optimized
matching

— 18

# FIG. 1

20

VB

FIG. 2

22

$P_{i-N}$

$P_{i+N}$

FIG. 3

FIG. 4

FIG. 5

Provide or collect
pre-operative images ── 302

Locate anatomical
reference in video image ── 304

Collect series of real
images near
pre-operative image ── 306

Compare video images
with pre-operative image
to find most similar ── 307

Register the pre-operative
image with the most
similar real image to
determine a
transformation matrix ── 308

# FIG. 6

FIG. 7

FIG. 8

EP 2 433 262 B1

Provide or collect CT scan images taken using markers — 350

Touch markers with tracking device to provide registration using phantom — 352

Capture CT image matching an image taken at a first endoscope position — 354

Adjust video image to better match the CT image — 356

Determine a rotation and translation matrix based on the adjustment to calibrate camera to tracker — 358

FIG. 9

**REFERENCES CITED IN THE DESCRIPTION**

**Patent documents cited in the description**

- JP 2003265408 A

- WO 2008065600 A2

**Non-patent literature cited in the description**

- **KENSAKU MORI et al.** Hybrid Bronchoscope Tracking Using a Magnetic Tracking Sensor and Image Registration. *Medical Image Computing and Computer-Assisted Intervention - MICCAI 2005, Lecture Notes in Computer Science,* 2005, vol. 3750, 543-550